# EUROPEAN PATENT SPECIFICATION

(11) **EP 4 162 900 B1**
(45) Date of publication and mention of the grant of the patent: **15.10.2025**
(21) Application number: 21914623.0
(22) Date of filing: 30.12.2021
(51) Int. Cl.: A61D 7/04, A61M 16/01

(54) **ANESTHESIA BREATHING DEVICE, ANESTHESIA BREATHING GAS PATH SYSTEM AND ANESTHESIA GAS PATH SYSTEM**
ANÄSTHESIEBEATMUNGSVORRICHTUNG, ANÄSTHESIEBEATMUNGSGASWEGSYSTEM UND ANÄSTHESIEGASWEGSYSTEM
DISPOSITIF DE RESPIRATION D'ANESTHÉSIE, SYSTÈME DE TRAJET DE GAZ RESPIRATOIRE D'ANESTHÉSIE ET SYSTÈME DE TRAJET DE GAZ D'ANESTHÉSIE

(30) Priority: 30.12.2020 WO PCT/CN2020/141403
(43) Date of publication of application: 12.04.2023
(73) Proprietor: Shenzhen Mindray Animal Medical Technology Co., Ltd., Shenzhen, Guangdong 518110 (CN)
(72) Inventor: CHAI, Haibo, Shenzhen, Guangdong 518110 (CN); SONG, Juntao, Shenzhen, Guangdong 518110 (CN)
(74) Representative: KIPA AB
(86) International application number: PCT/CN2021/143055
(87) International publication number: WO 2022/143892

(56) References cited:
- EP-A2- 0 919 253
- CN-A- 105 457 138
- CN-A- 109 157 300
- CN-A- 109 260 557
- CN-A- 111 084 925
- CN-U- 210 277 915
- US-A- 5 692 494
- US-A1- 2009 056 720
- US-A1- 2011 088 694
- US-A1- 2012 031 402
- US-A1- 2013 306 067
- US-A1- 2020 353 201
- US-B1- 6 571 792

## Description

### TECHNICAL FIELD

The disclosure relates to the field of medical instruments, and in particular, to a structure for achieving anesthesia function or anesthesia respiration function.

### BACKGROUND

In veterinary medical instruments, a veterinary anesthesia machine and a veterinary anesthesia ventilator are usually two separate devices. The anesthesia machine mainly supplies anesthetic gas to a subject (animal) to meet anesthesia requirements of certain operations. The anesthesia ventilator can not only deliver anesthetic gas, but also help a subject breathe. Usually, the two devices are simply placed side by side and used in combination, and a large number of pipelines are connected externally, which not only makes connections difficult and is poor in ease of use, but also makes exposed pipelines prone to touch by accident. In addition, when placed together, the two separate devices have a relatively large overall volume and takes up an excessive space in an operating room.

In patent application publication EP 0 919 253 A2, a Modular anesthesia respiratory system is disclosed. An anesthesia system is disclosed wherein the respiratory system is fitted with interchangeable patient circuit modules that allows a generic fitting in the machine to have affixed, hereto, a module that has ports suitable for connection to various patient breathing circuits. As such, the anesthesia machine can have the patient circuit modules changed.

In patent application publication US 2013/306067A1, a ceiling-mounted supply unit is disclosed for fastening a medical device on the ceiling-mounted supply unit, which includes a vertical beam, an extension arm, to which the vertical beam is fastened especially with a hinge, for moving, especially pivoting the vertical beam and electrical and/or pneumatic supply lines with electrical and/or pneumatic connections. A medical device is integrated in the vertical beam.

In patent application publication CN 111 084 925 A, an anesthesia machine is disclosed providing a positioning component, which can enable a user to determine whether a fluid delivery pipeline module is installed in place after placing the fluid delivery pipeline module into an installation channel preventing the fluid delivery pipeline module from failing a situation where gas leakage is caused by installation in place, which in turn affects the performance of the equipment.

In patent application publication US 2012/031402A1, a respiratory anesthetic aggregate devised for coupling to a breathing apparatus is disclosed, which has an anesthetic gasifier unit that has a breathing gas input port connected to a first point in an inspiratory patient gas delivery path of a breathing apparatus, and a fresh gas output port connected to a second point in the patient gas delivery path downstream the first point. The inspiratory patient gas delivery path is connected via a mainstream connection between the first and second point. Thereby the anesthetic gasifier unit is connected in a sidestream configuration to the patient gas delivery path for adding the gasified anesthetic agent in a desired concentration to the patient gas delivery path at the second point. When the aggregate is coupled to a breathing apparatus, a substantially volume neutral delivery of anesthetic agent is provided a to the breathing apparatus.

### SUMMARY

The present invention is defined by the appended claims only, in particular by the scope of appended independent claim 1. References to "embodiments" throughout the description which are not under the scope of the appended claims merely represents possible exemplary executions and are therefore not part of the present invention.

The disclosure provides a novel integrated veterinary anesthesia respiration apparatus, an anesthesia respiration gas path system and an anesthetic gas path system.

Based on the foregoing objective, an example of the disclosure provides an integrated veterinary anesthesia respiration apparatus, including:
an anesthesia main machine configured to supply an anesthetic gas and having a first interface group and a second interface group;
a ventilator configured to help a subject breathe, capable of being detachably docked with the anesthesia main machine and having a third interface group capable of being detachably docked with the first interface group;
a first breathing circuit mechanism having a fourth interface group capable of being detachably docked with the second interface group;
a second breathing circuit mechanism having a fifth interface group capable of being detachably docked with the second interface group;
and a top plate having a blocking structure capable of blocking the first interface group of the anesthesia main machine;
wherein the anesthesia main machine is detachably connected to the ventilator and the first breathing circuit mechanism to form an anesthesia respiration gas path system, so as to output the anesthetic gas to the subject and help the subject breathe; or the anesthesia main machine is detachably connected to the top plate and the second breathing circuit mechanism to form an anesthetic gas path system, so as to output the anesthetic gas to the subject.

In an example, in the anesthesia respiration gas path system, the anesthesia main machine and the ventilator are superposed one above another, and the ventilator is located above the anesthesia main machine.

In an example, the anesthesia main machine has a main machine housing, the first interface group is arranged on an upper end of the main machine housing, the ventilator has a ventilator housing, and the third interface group is arranged on a lower end of the ventilator housing.

In an example, in the anesthesia respiration gas path system, the first breathing circuit mechanism is located on a side of the anesthesia main machine.

In an example, the second interface group is arranged below a side of the main machine housing, the first breathing circuit mechanism has a first breathing circuit mounting base, and the fourth interface group is arranged on a side wall of the first breathing circuit mounting base opposite to the anesthesia main machine.

In an example, in the anesthetic gas path system, the anesthesia main machine and the second breathing circuit mechanism are arranged one above another, and the anesthesia main machine is arranged above the second breathing circuit mechanism.

In an example, the anesthesia main machine has a main machine housing, the first interface group is arranged on an upper end of the main machine housing, and the top plate covers the upper end of the main machine housing to block the first interface group.

In an example, the second interface group is arranged below a side of the main machine housing, the second breathing circuit mechanism has a second breathing circuit mounting base, and the fifth interface group is arranged on a side wall of the second breathing circuit mounting base opposite to the anesthesia main machine.

In an example, the anesthesia main machine is provided with a volatilization tank for generating the anesthetic gas and a flowmeter for measuring a gas flow.

In an example, the first breathing circuit mechanism is provided with a bellows, a carbon dioxide absorber, a manually/mechanically-controlled valve assembly, and an APL valve.

In an example, the second breathing circuit mechanism is provided with a carbon dioxide absorber and an APL valve.

In an example, the first interface group of the anesthesia main machine comprises a first sampling interface, a first driving gas interface, and a first exhaust emission interface, and the third interface group of the ventilator comprises a second sampling interface docked with the first sampling interface, a second driving gas interface docked with the first driving gas interface, and a second exhaust emission interface docked with the first exhaust emission interface.

Based on the foregoing objective, an embodiment of the disclosure provides an anesthesia respiration gas path system, including:
an anesthesia main machine configured to supply an anesthetic gas, wherein the anesthesia main machine is provided with a main machine housing and a volatilization tank for volatilizing an anesthetic drug, a first anesthetic gas passage is provided inside the main machine housing, the first anesthetic gas passage communicates with the volatilization tank, the first anesthetic gas passage is provided with a gas source interface for an external gas source to enter and a fresh gas interface for outputting an anesthetic gas mixture containing the anesthetic gas, and the gas source interface and the fresh gas interface are arranged on the main machine housing;
a ventilator having a ventilator housing, wherein a second driving gas channel and a second exhaust passage for connecting to an exhaust treatment device are provided inside the ventilator housing, the second driving gas channel has a third driving gas interface for outputting a driving gas, the second exhaust passage has a third exhaust emission interface for emitting exhaust, and the third driving gas interface and the third exhaust emission interface are arranged on the ventilator housing; and
a first breathing circuit mechanism having a first breathing circuit mounting base and a bellows, wherein the bellows is connected to the first breathing circuit mounting base, a second anesthetic gas passage and a third exhaust passage are provided inside the first breathing circuit mounting base, the second anesthetic gas passage has a second fresh gas interface for the anesthetic gas mixture to enter and a user interface for leading the anesthetic gas mixture to a subject, the third exhaust passage has a fourth exhaust emission interface for emitting exhaust, the second fresh gas interface, the user interface and the fourth exhaust emission interface are all arranged on the first breathing circuit mounting base, and the fresh gas interface fits and communicates with the second fresh gas interface, so as to deliver the anesthetic gas mixture into the user interface;
wherein a first driving gas passage and/or a first exhaust passage are/is provided inside the main machine housing, the first driving gas passage has a first driving gas interface and a second driving gas interface which are arranged on the main machine housing, the first driving gas interface is configured for a driving gas to enter, the second driving gas interface is configured to output the driving gas, the first exhaust passage has a first exhaust emission interface and a second exhaust emission interface which are arranged on the main machine housing, the second exhaust emission interface is configured for the exhaust in the first breathing circuit mechanism to enter, and the first exhaust emission interface is configured to deliver the exhaust to the third exhaust emission interface;
the third driving gas interface communicates with the first driving gas interface to input the driving gas into the first driving gas passage, and the second driving gas interface is configured to communicate with the bellows to drive the bellows to operate; and
the fourth exhaust emission interface communicates with the second exhaust emission interface, and the first exhaust emission interface communicates with the third exhaust emission interface, to discharge the exhaust into the exhaust treatment device.

In an embodiment, the ventilator and the anesthesia main machine are superposed one above another, and are detachably connected to each other, the first driving gas interface is arranged on a top end of the main machine housing, and the third driving gas interface is arranged on a bottom end of the ventilator housing.

In an embodiment, the first breathing circuit mechanism and the anesthesia main machine are arranged side by side, and are detachably connected to each other, the fresh gas interface and/or the second driving gas interface are/is arranged on a side of the main machine housing, and the second fresh gas interface is arranged on a side of the first breathing circuit mounting base opposite to the main machine housing.

In an embodiment, the ventilator and the anesthesia main machine are superposed one above another, and are detachably connected to each other, the first exhaust emission interface is arranged on a top end of the main machine housing, and the third exhaust emission interface is arranged on a bottom end of the ventilator housing.

In an embodiment, the first breathing circuit mechanism and the anesthesia main machine are arranged side by side, and are detachably connected to each other, the fresh gas interface and/or the second exhaust emission interface are/is arranged on a side of the main machine housing, and the fourth exhaust emission interface is arranged on a side of the first breathing circuit mounting base opposite to the main machine housing.

In an embodiment, the fresh gas interface fits and communicates with the second fresh gas interface in a detachable insertion manner;
and/or the third driving gas interface fits and communicates with the first driving gas interface in a detachable insertion manner;
and/or the fourth exhaust emission interface fits and communicates with the second exhaust emission interface in a detachable insertion manner;
and/or the first exhaust emission interface fits and communicates with the third exhaust emission interface in a detachable insertion manner;
and/or the second driving gas interface fits and communicates with the bellows in a detachable insertion manner.

In an embodiment, the anesthesia respiration gas path system further includes a top plate which is disposed above the ventilator housing.

Based on the foregoing objective, an embodiment of the disclosure provides an anesthetic gas path system, including:
an anesthesia main machine configured to supply an anesthetic gas, wherein the anesthesia main machine is provided with a main machine housing and a volatilization tank for volatilizing an anesthetic drug, a first anesthetic gas passage is provided inside the main machine housing, the first anesthetic gas passage communicates with the volatilization tank, the first anesthetic gas passage is provided with a gas source interface for an external gas source to enter and a fresh gas interface for outputting an anesthetic gas mixture containing the anesthetic gas, and the gas source interface and the fresh gas interface are arranged on the main machine housing; and
a second breathing circuit mechanism having a second breathing circuit mounting base, wherein a second anesthetic gas passage is provided in the second breathing circuit mounting base, the second anesthetic gas passage has a second fresh gas interface for the anesthetic gas mixture to enter and a user interface for leading the anesthetic gas mixture to a subject, the second fresh gas interface and the user interface are arranged on the second breathing circuit mounting base, and the fresh gas interface is in detachable insertion fit and communication with the second fresh gas interface, so as to deliver the anesthetic gas mixture into the user interface.

In an embodiment, a first driving gas passage and/or a first exhaust passage are/is provided inside the main machine housing, the first driving gas passage has a first driving gas interface and a second driving gas interface which are arranged on the main machine housing, the first driving gas interface serves for a driving gas from a ventilator to enter, the second driving gas interface outputs the driving gas, the first exhaust passage has a first exhaust emission interface and a second exhaust emission interface which are arranged on the main machine housing, the second exhaust emission interface serves for the exhaust in the first breathing circuit mechanism to enter, and the first exhaust emission interface is configured for delivering the exhaust to the third exhaust emission interface of the ventilator.

In an embodiment, the anesthetic gas path system further includes a blocking member, wherein the blocking member blocks the first driving gas interface, the second driving gas interface, the first exhaust emission interface, and/or the second exhaust emission interface.

In an embodiment, the second breathing circuit mechanism and the anesthesia main machine are arranged side by side, and are detachably connected to each other, the fresh gas interface is arranged on a side of the main machine housing, and the second fresh gas interface is arranged on a side of the second breathing circuit mounting base opposite to the main machine housing.

Based on the foregoing objective, an embodiment of the disclosure provides an integrated veterinary anesthesia respiration apparatus, including:
an anesthesia main machine configured to supply an anesthetic gas and having a first interface group and a second interface group;
a ventilator configured to help a subject breathe, capable of being detachably docked with the anesthesia main machine and having a third interface group capable of being detachably docked with the first interface group;
a first breathing circuit mechanism having a fourth interface group capable of being detachably docked with the second interface group;
and a second breathing circuit mechanism having a fifth interface group capable of being detachably docked with the second interface group;
wherein the anesthesia main machine is detachably connected to the ventilator and the first breathing circuit mechanism to form an anesthesia respiration gas path system, so as to output the anesthetic gas to the subject and help the subject breathe; or the anesthesia main machine is detachably connected to a top plate and the second breathing circuit mechanism to form an anesthetic gas path system, so as to output the anesthetic gas to the subject.

In the integrated veterinary anesthesia respiration apparatus according to the foregoing embodiment, an anesthesia main machine, a ventilator, a first breathing circuit mechanism, a top plate, and a second breathing circuit mechanism are provided. The anesthesia main machine, the ventilator, the first breathing circuit mechanism, the top plate, and the second breathing circuit mechanism may be selectively combined. For example, the anesthesia main machine is detachably connected to the ventilator and the first breathing circuit mechanism to form an anesthesia respiration gas path system, so as to output an anesthetic gas to a subject and help the subject breathe. Alternatively, the anesthesia main machine is detachably connected to the top plate and the second breathing circuit mechanism to form an anesthetic gas path system, so as to output the anesthetic gas to the subject.

A user may assemble the anesthesia respiration gas path system or the anesthetic gas path system as required. The assembled apparatus as a whole has a small footprint. In addition, all gas paths are docked with each other through external interfaces, requirements for gas path connection can be met without pipelines or with only a small number of pipelines, thereby reducing various potential safety hazards caused by excessive exposed pipelines.

The anesthesia respiration gas path system according to the foregoing embodiment includes an anesthesia main machine, a ventilator, and a first breathing circuit mechanism. The anesthesia main machine has a main machine housing and a volatilization tank for volatilizing an anesthetic drug, and at least one of a first anesthetic gas passage, a first driving gas passage and a first exhaust passage is provided inside the main machine housing. The first anesthetic gas passage communicates with a fresh gas interface of the first breathing circuit mechanism to deliver an anesthetic gas mixture to a user. The first driving gas passage may be configured to enable a third driving gas interface of the ventilator to communicate with a bellows of the first breathing circuit mechanism to drive the bellows to operate. The first exhaust passage is configured to enable a third exhaust emission interface of the ventilator to communicate with a fourth exhaust emission interface of the first breathing circuit mechanism to discharge exhaust. In this structure, at least one of the first anesthetic gas passage, the first driving gas passage and the first exhaust passage is provided inside the main machine housing, which avoids externally arranged pipelines and prevents the pipelines from being entangled to influence user's actions. In addition, this structure with built-in passages also makes it easier to assemble the entire system.

The anesthetic gas path system according to the foregoing embodiment includes an anesthesia main machine and a second breathing circuit mechanism. The anesthesia main machine has a main machine housing and a volatilization tank for volatilizing an anesthetic drug, and a first anesthetic gas passage is provided inside the main machine housing. The first anesthetic gas passage has a fresh gas interface for outputting an anesthetic gas mixture containing an anesthetic gas, and the fresh gas interface is arranged on the main machine housing. The second breathing circuit mechanism has a second breathing circuit mounting base, a second anesthetic gas passage is provided in the second breathing circuit mounting base, the second anesthetic gas passage has a second fresh gas interface for the anesthetic gas mixture to enter, the second fresh gas interface is arranged on the second breathing circuit mounting base, and the fresh gas interface fits and communicates with the second fresh gas interface in a detachable insertion manner, without excessively long connecting pipelines, which avoids externally arranged pipelines and prevents the pipelines from being entangled to influence user's actions. In addition, this structure with built-in passages also makes it easier to assemble the entire system.

### BRIEF DESCRIPTION OF THE DRAWINGS

FIG. 1 is a schematic structural diagram of an anesthesia main machine in an embodiment of the disclosure;
FIG. 2 is a schematic structural diagram of a ventilator in an embodiment of the disclosure;
FIG. 3 is a schematic structural diagram of a top plate in an embodiment of the disclosure;
FIG. 4 is a schematic structural diagram of a first breathing circuit mechanism in an embodiment of the disclosure;
FIG. 5 is a schematic structural diagram of a second breathing circuit mechanism in an embodiment of the disclosure;
FIG. 6 is a schematic structural diagram of an anesthesia respiration gas path system in an embodiment of the disclosure;
FIG. 7 is a schematic structural diagram of an anesthetic gas path system in an embodiment of the disclosure;
FIG. 8 is a schematic diagram of gas path connection of an anesthesia respiration gas path system in an embodiment of the disclosure; and
FIG. 9 is a schematic diagram of gas path connection of an anesthetic gas path system in an embodiment of the disclosure.

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, the detailed description of the involved operations is not necessary, and the involved operations can be thoroughly understood according to the description in the specification and general technical knowledge in the art.

In addition, the characteristics, operations, or features described in the specification may be combined in any appropriate manner to form various implementations. In addition, the steps or actions in the method description may also be exchanged or adjusted in order in a way that is obvious to persons skilled in the art. Therefore, the various orders in the specification and the accompanying drawings are merely for the purpose of clear description of a certain embodiment and are not meant to be a necessary order unless it is otherwise stated that a certain order must be followed.

The serial numbers themselves for the components herein, for example, "first" and "second", are merely used to distinguish the described objects, and do not have any sequential or technical meaning. Moreover, as used in the disclosure, "connection" or "coupling", unless otherwise stated, includes both direct and indirect connections (couplings).

An embodiment of the disclosure provides an integrated veterinary anesthesia respiration apparatus (for ease of description, hereinafter referred to as an integrated anesthesia ventilator). The integrated anesthesia ventilator includes a plurality of components capable of being combined in a detachable manner, which may be assembled into an anesthesia respiration gas path system to output an anesthetic gas to a subject (such as an animal) and help the subject breathe, or may be assembled into an anesthetic gas path system to output the anesthetic gas to the subject, according to user's requirements.

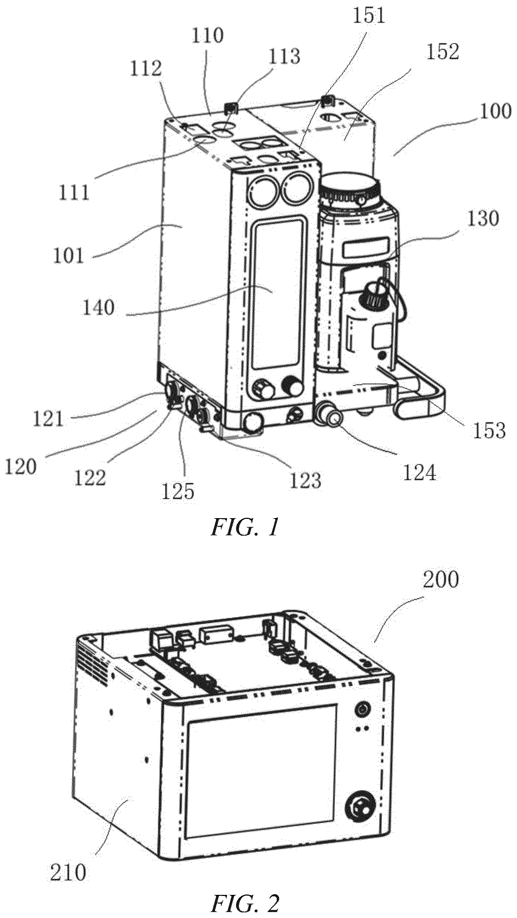

Specifically, referring to FIGS. 1-5, the integrated anesthesia ventilator includes an anesthesia main machine 100, a ventilator 200, a top plate 300, a first breathing circuit mechanism 400, and a second breathing circuit mechanism 500.

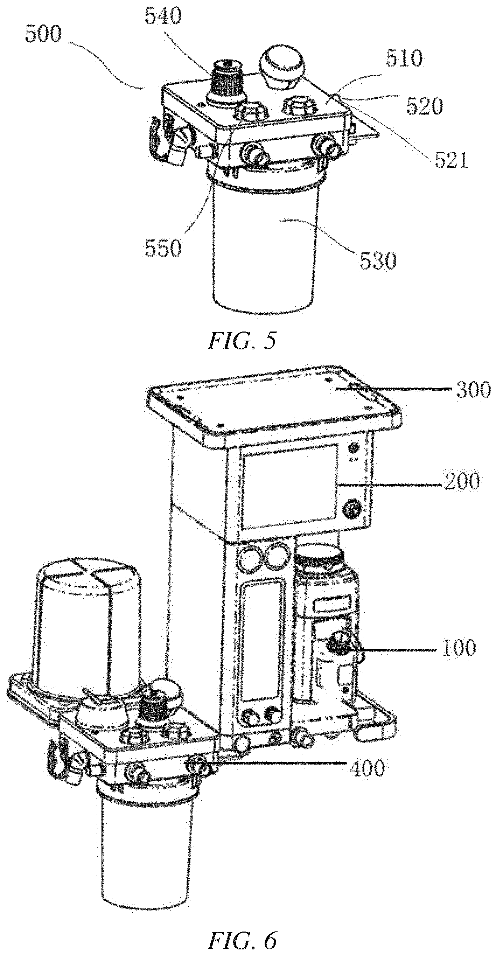

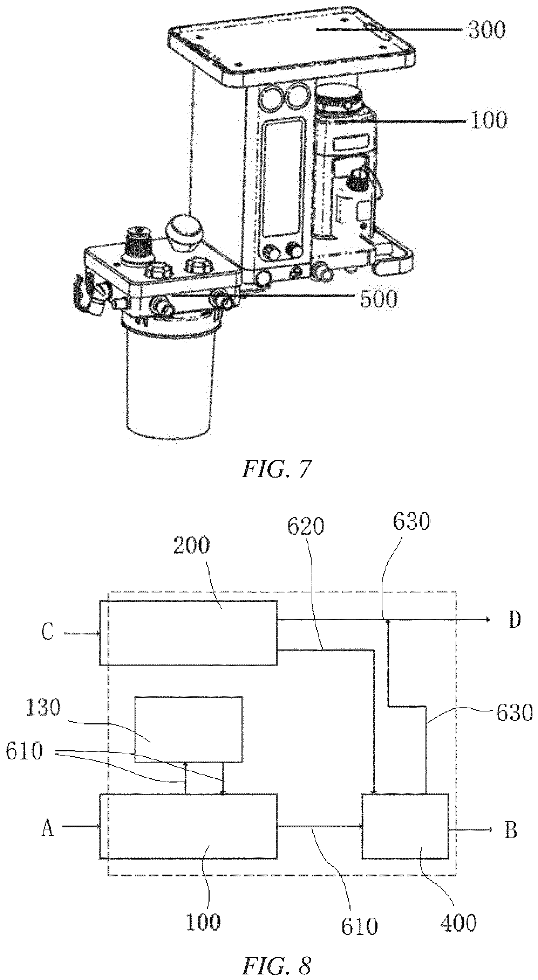

Referring to FIG. 6, the anesthesia main machine 100 is detachably connected to the ventilator 200 and the first breathing circuit mechanism 400 to form an anesthesia respiration gas path system, so as to output an anesthetic gas to a subject and help the subject breathe. Alternatively, referring to FIG. 7, the anesthesia main machine 100 is detachably connected to the top plate 300 and the second breathing circuit mechanism 500 to form an anesthetic gas path system, so as to output the anesthetic gas to the subject.

The anesthesia respiration gas path system has a complete anesthetic gas path and breathing circuit. In an embodiment, when oxygen supply is insufficient or interrupted, nitrous oxide supply is automatically cut off. In use, concentrations of O2 and N2O in the breathing circuit are monitored by the integrated anesthesia ventilator or other monitoring systems, which can more accurately measure current operation of the anesthesia machine.

The anesthetic gas path system is configured to mainly supply an anesthetic gas to a subject, and the anesthetic gas may be led to a combined gas path device (such as another breathing circuit) connected to a patient, so as to deliver the anesthetic gas to the patient.

Further, the anesthesia main machine 100 is configured to supply an anesthetic gas, and has a corresponding anesthetic gas path. For example, referring to FIG. 1, the anesthesia main machine 100 is provided with a volatilization tank 130 for generating an anesthetic gas and a flowmeter 140 for measuring a gas flow.

The volatilization tank 130 may also be referred to as an evaporation tank, an anesthesia vaporizer, or the like. The volatilization tank 130 is configured to turn the anesthetic drug into an evaporative gas based on variations in temperature and a heat source in surrounding environment, and through a certain amount of carrier gas, a part of the gas carries a saturated anesthetic gas and becomes a gas flow of anesthetic vapor with a certain concentration, which directly enters the anesthesia circuit. The flowmeter 140 is configured to monitor corresponding gas flows, such as O₂, N₂O, fresh gas and gas mixture. In an embodiment, the flowmeter 140 may include two reading flow tubes with units of L/min and mL/min (or a flow tube with a unit less than 2 L/min), so as to facilitate implementation of low flow anesthesia.

The anesthesia main machine 100 has a main machine housing 101, and the main machine housing 101 is provided with a first interface group 110 and a second interface group 120. The first interface group 110 and the second interface group 120 communicate with the anesthetic gas path inside the anesthesia main machine 100 to facilitate connecting the anesthetic gas path with other components. The first interface group 110 and the second interface group 120 may have several interfaces to implement flow of different gas sources.

Specifically, in an embodiment, referring to FIG. 1, the first interface group 110 of the anesthesia main machine 100 includes a first driving gas interface 111, a first sampling interface 112, a first exhaust emission interface 113, etc. The second interface group 120 of the anesthesia main machine 100 includes a second sampling interface 122, a second driving gas interface 121, a fresh gas interface 123, an independent auxiliary common gas outlet (ACGO) interface 124, a second exhaust emission interface 125, etc.

Referring to FIGS. 2 and 6, the ventilator 200 is configured to help the subject breathe. The ventilator 200 can implement mechanical ventilation to assist and control a patient's breathing, improve the patient's oxygenation and ventilation, reduce work done by respiratory muscles, and support circulatory function, etc. The ventilator 200 has a structure that is capable of being detachably docked with the anesthesia main machine 100.

Specifically, the ventilator 200 has a third interface group (not shown) that is capable of being detachably docked with the first interface group 110. The third interface group has several different interfaces which communicate with an internal gas path of the ventilator 200 and which serve to make the ventilator 200 communicate with a related gas path of the anesthesia machine.

In an embodiment, the third interface group of the ventilator 200 includes a third sampling interface docked with the first sampling interface, a third driving gas interface docked with the first driving gas interface 111, and a third exhaust emission interface docked with the first exhaust emission interface 113. The third sampling interface, the third driving gas interface, and the third exhaust emission interface are not shown in the figures.

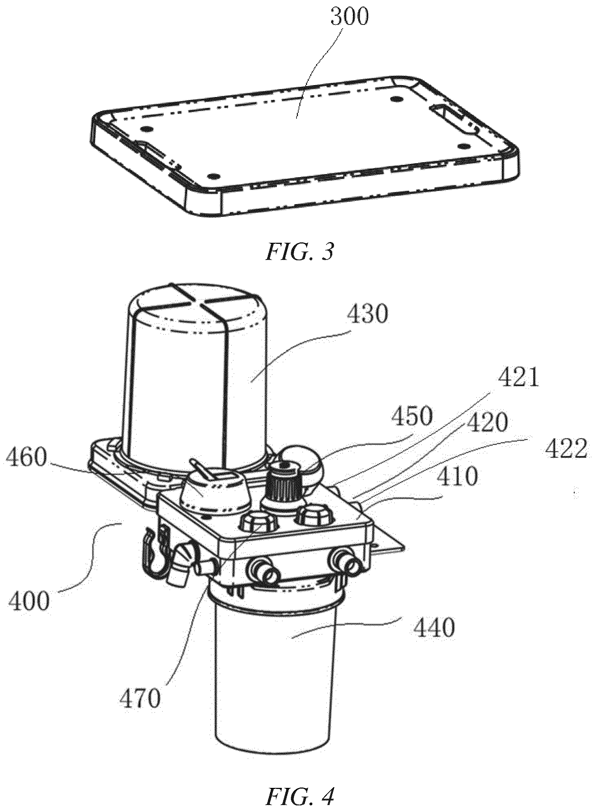

Further, referring to FIG. 6, the top plate 300 may be mounted on the ventilator 200, and may be used as a receiving tray for receiving and placing some articles thereon. Referring to FIGS. 3 and 7, the top plate 300 has a blocking structure capable of blocking the first interface group 110 of the anesthesia main machine 100. The top plate 300 may be mounted on the anesthesia main machine 100 and configured to block the first interface group 110 of the anesthesia main machine 100. The first interface group 110 is originally configured for docking with the ventilator 200. The anesthetic gas path system shown in FIG. 7 includes no ventilator 200, and thus the top plate 300 is used to seal the first interface group 110. Certainly, in the embodiment shown in FIG. 7, the top plate 300 may also be used as a receiving tray.

Referring to FIGS. 4 and 6, the first breathing circuit mechanism 400 has a gas path adapted to the anesthesia respiration gas path system. The first breathing circuit mechanism 400 has a fourth interface group 420 for detachably docking with the second interface group 120 when an anesthesia respiration gas path system is formed, so as to make the first breathing circuit mechanism 400 communicate with the anesthesia main machine 100.

Referring to FIGS. 4 and 6, in an embodiment, the first breathing circuit mechanism 400 is provided with a bellows 430, a carbon dioxide absorber 440 (such as a soda lime tank), a manually/mechanically-controlled valve assembly 460, an adjustable pressure limiting (APL) valve 450 (also referred to as a gas escape valve or a pressure reducing valve), a valve cover 470, and other related structures. The manually/mechanically-controlled valve assembly 460 makes it convenient for a user to choose manual control or mechanical control.

Referring to FIGS. 5 and 7, the second breathing circuit mechanism 500 has a gas path adapted to the anesthetic gas path system. The second breathing circuit mechanism 500 has a fifth interface group 520 for detachably docking with the fifth interface group 520 when an anesthetic gas path system is formed, so as to make the second breathing circuit mechanism 500 communicate with the anesthesia main machine 100.

Referring to FIGS. 5 and 7, in an embodiment, the second breathing circuit mechanism 500 is provided with a carbon dioxide absorber 530, an adjustable pressure limiting (APL) valve 540 (also referred to as a gas escape valve or a pressure reducing valve), a valve cover 550, and other related structures.

Further, in an embodiment, referring to FIG. 6, in the anesthesia respiration gas path system, the anesthesia main machine 100 and the ventilator 200 are superposed one above another, and the ventilator 200 is located above the anesthesia main machine 100. This vertical superposition has a small horizontally occupied space and more compact structure, which is more suitable for a specific situation where a community pet hospital has a constrained space, and can better meet use by clinical users.

Referring to FIGS. 1 and 6, in an embodiment, the first interface group 110 is arranged on an upper end of the main machine housing 101, the ventilator 200 has a ventilator housing 210, and the third interface group is arranged on a lower end of the ventilator housing 210.

To make better use of vertical space, referring to FIGS. 1, 4 and 6, in the anesthesia respiration gas path system, the first breathing circuit mechanism 400 is located on a side of the anesthesia main machine 100. The second interface group 120 is arranged below a side of the main machine housing 101, the first breathing circuit mechanism 400 has a first breathing circuit mounting base 410, and the fourth interface group 420 is arranged on a side wall of the first breathing circuit mounting base 410 opposite to the anesthesia main machine 100.

In another embodiment, referring to FIGS. 1, 5 and 7, in the anesthetic gas path system, the anesthesia main machine 100 and the second breathing circuit mechanism 500 may also be arranged one above another, and the anesthesia main machine 100 is arranged above the second breathing circuit mechanism 500. For example, the anesthesia main machine 100 has a main machine housing 101, the first interface group 110 is arranged on an upper end of the main machine housing 101, and the top plate 300 covers the upper end of the main machine housing 101 to block the first interface group 110.

The second interface group 120 is arranged below a side of the main machine housing 101, the second breathing circuit mechanism 500 has a second breathing circuit mounting base 510, and the fifth interface group 520 is arranged on a side wall of the second breathing circuit mounting base 510 opposite to the anesthesia main machine 100.

On the other hand, to achieve two functions of supplying an anesthetic gas and helping a subject breathe, an anesthesia machine and a ventilator are usually simply placed side by side and used in combination. A large number of external long pipelines are provided between the anesthesia machine and the ventilator, which causes a troublesome docking. And these external long pipelines also occupy plenty of space and are often entangled together, or even worse, trip a user. Especially in animal medical environment, because animals tend to move, it is more likely for animals to accidentally touch these pipelines, which not only causes harm to the animals, but also may cause damage to connections of devices, resulting in leakage of anesthetic gas and medical accidents.

In view of this, an embodiment of the disclosure provides an anesthesia respiration gas path system which can output an anesthetic gas to a subject (such as an animal) and help the subject breathe.

Referring to FIGS. 1, 2, 4, 6 and 8, in an embodiment, the anesthesia respiration gas path system includes components such as an anesthesia main machine 100, a ventilator 200, and a first breathing circuit mechanism 400. As shown in FIG. 8, an anesthetic gas path 610 is provided between the anesthesia main machine 100 and the first breathing circuit mechanism 400 for conveying an anesthetic gas mixture. A driving gas path 620 and an exhaust emission path 630 are provided between the ventilator 200 and the first breathing circuit mechanism 400. The driving gas path 620 is configured to convey a driving gas, in order to drive the bellows 430 to operate. The exhaust emission path 630 is configured to collect and discharge exhaust. The driving gas path 620 and the exhaust emission path 630 may communicate with the first breathing circuit mechanism 400 directly by the ventilator 200, or may make the ventilator 200 communicate with the first breathing circuit mechanism 400 by using the anesthesia main machine 100 as an intermediate connecting member.

Referring to FIGS. 1, 6 and 8, the anesthesia main machine 100 is configured to supply an anesthetic gas. The anesthesia main machine 100 has a main machine housing 101 and a volatilization tank 130 for volatilizing an anesthetic drug. A first anesthetic gas passage is provided inside the main machine housing 101, and is configured to supply the anesthetic gas. The first anesthetic gas passage communicates with the volatilization tank 130, and the anesthetic gas volatilized from the volatilization tank 130 can enter the first anesthetic gas channel.

The first anesthetic gas passage has a gas source interface (not shown) for an external gas source A to enter and a fresh gas interface 123 for outputting an anesthetic gas mixture with the anesthetic gas mixed, and the gas source interface and the fresh gas interface 123 are arranged on the main machine housing 101. The external gas source A may be external air, or may be a compressed-oxygen cylinder, a compressed-air cylinder, an oxygen generator, a wall-mounted gas source (oxygen or air), etc. In an embodiment, the anesthetic gas mixture may be a mixture of an anesthetic gas and at least one of air and oxygen. In some embodiments, for example, the anesthetic gas mixture may also have N₂O or the like mixed therein.

In addition, in some embodiments, the anesthesia main machine 100 may be further provided with components such as a flowmeter 140 and a pressure meter. The flowmeter 140 is configured to monitor a corresponding gas flow. In an embodiment, the flowmeter 140 may include two reading flow tubes with units of L/min and mL/min (or a flow tube with a unit less than 2 L/min), so as to facilitate implementation of low flow anesthesia.

In an embodiment, the gas source interface may be arranged on a back of the main machine housing 101, that is, a side that faces away from the volatilization tank 130 and the flowmeter 130.

In an embodiment, the main machine housing 101 is provided with a placement position for placing the volatilization tank 130. Preferably, the placement position includes a first side wall 151, a second side wall 152, and a placement table 153. The first side wall 151 and the second side wall 152 are connected and arranged perpendicular to each other. The placement table 153 is arranged perpendicular to the first side wall 151 and the second side wall 152. The volatilization tank 130 is disposed on the placement table 153.

The ventilator 200 is configured to help a subject breathe. The ventilator 200 can implement mechanical ventilation to assist and control a patient's breathing, improve the patient's oxygenation and ventilation, reduce work done by ventilatory muscles, and support circulatory function, etc. In an embodiment, the ventilator 200 includes a turbine or other driving devices configured to drive an external gas source C to enter the entire gas path system, so as to output a driving gas to the first breathing circuit mechanism 400 or the subsequent second breathing circuit mechanism 500, thereby driving the anesthetic gas to enter an animal.

Referring to FIGS. 2, 6 and 8, the ventilator 200 has a ventilator housing 210. A second driving gas channel and a second exhaust passage for connecting to an exhaust treatment device D are provided inside the ventilator housing 210. The second driving gas channel is configured to input a driving gas, to drive a bellows 430 (or referred to as a collapsible bag) of the first breathing circuit mechanism 400 to deform, thereby assisting a user to breathe. The second exhaust passage communicates with the first breathing circuit mechanism 400, and discharges exhaust with a residual anesthetic gas from the first breathing circuit mechanism 400. The second exhaust passage may be driven by the ventilator 200, to finally discharge exhaust to the exhaust treatment device D for treatment.

The second driving gas channel has a third driving gas interface (located below the ventilator 200 in the figure, and not shown) for outputting a driving gas. The second exhaust passage has a third exhaust emission interface (located below the ventilator 200 in the figure, and not shown) for emitting exhaust, and the third driving gas interface and the third exhaust emission interface are arranged on the ventilator housing 210 to facilitate docking.

Referring to FIGS. 4, 6 and 8, the first breathing circuit mechanism 400 has a first breathing circuit mounting base 410 and a bellows 430 (or referred to as a collapsible bag). The bellows 430 is connected to the first breathing circuit mounting base 410. A second anesthetic gas passage and a third exhaust passage are provided inside the first breathing circuit mounting base 410. The second anesthetic gas passage has a second fresh gas interface 421 for an anesthetic gas mixture to enter and a user interface B for leading the anesthetic gas mixture to a user. The fresh gas interface 123 fits and communicates with the second fresh gas interface 421, so as to deliver the anesthetic gas mixture into the user interface B for inhaling by the user.

The third exhaust passage has a fourth exhaust emission interface 422 for emitting exhaust. The second fresh gas interface 421, the user interface B and the fourth exhaust emission interface 422 are all arranged on the first breathing circuit mounting base 410 to facilitate docking.

In addition, to reduce exposed pipelines, a first driving gas passage and/or a first exhaust passage are/is provided inside the main machine housing 101. The first driving gas passage has a first driving gas interface 111 and a second driving gas interface 121 which are arranged on the main machine housing 101. The first driving gas interface 111 serves for a driving gas to enter, and the second driving gas interface 121 outputs the driving gas. A third driving gas interface of the ventilator 200 communicates with the first driving gas interface 111 to input the driving gas into the first driving gas passage. The second driving gas interface 121 of the anesthesia main machine 100 is configured to communicate with the bellows 430 to drive the bellows 430 to operate.

The first exhaust passage has a first exhaust emission interface 113 and a second exhaust emission interface 125 which are arranged on the main machine housing 101. The second exhaust emission interface 125 serves for exhaust in the first breathing circuit mechanism 400 to enter, and the first exhaust emission interface 113 is configured to deliver the exhaust to the third exhaust emission interface. The fourth exhaust emission interface 422 of the first breathing circuit mechanism 400 communicates with the second exhaust emission interface 125, and the first exhaust emission interface 113 communicates with the third exhaust emission interface, to discharge the exhaust into the exhaust treatment device D under control of the ventilator 200.

Where one of the first driving gas passage and the first exhaust passage is provided inside the main machine housing 101, the other passage may be provided as an external pipeline, so as to enable the ventilator 200 to communicate with the first breathing circuit mechanism 400.

In the anesthesia respiration gas path system, at least one of the first anesthetic gas passage, the first driving gas passage and the first exhaust passage is provided inside the main machine housing 101, which avoids externally arranged pipelines and prevents pipelines from being entangled to influence user's actions. In addition, this structure with built-in channels also makes it easier to assemble the entire system.

In an embodiment, to further reduce the number of external pipelines, some or all of the interfaces between the anesthesia main machine 100, the ventilator 200 and the first breathing circuit mechanism 400 may be provided as a detachable insertion fit structure. When the anesthesia respiration gas path system is assembled, the interfaces may be quickly docked. In addition, influences of external pipelines between the anesthesia main machine 100, the ventilator 200 and the first breathing circuit mechanism 400 are eliminated, risk of entanglement is reduced and influence of pipelines to user's actions are prevented.

For example, referring to FIGS. 1, 2, 4 and 6, in an embodiment, the fresh gas interface 123 fits and communicates with the second fresh gas interface 421 in a detachable insertion manner;
and/or the third driving gas interface fits and communicates with the first driving gas interface 111 in a detachable insertion manner;
and/or the fourth exhaust emission interface 422 fits and communicates with the second exhaust emission interface 125 in a detachable insertion manner;
and/or the first exhaust emission interface 113 fits and communicates with the third exhaust emission interface in a detachable insertion manner;
and/or the second driving gas interface 121 fits and communicates with the bellows 430 in a detachable insertion manner.

The detachable insertion fit means that two interfaces may communicate with each other by means of a detachable insertion structure instead of a common pipeline, and the interfaces communicate with each other without a pipeline or with a quite short pipeline therebetween, so as to reduce long pipelines.

Further, referring to FIG. 6, in an embodiment, the ventilator 200 and the anesthesia main machine 100 are superposed one above another. The ventilator 200 has a structure that is capable of being detachably docked with the anesthesia main machine 100 to implement detachable connection. This vertical superposition has a small horizontally occupied space and a more compact structure, which is more suitable for a specific situation where a community pet hospital has a constrained space, and can better meet use by clinical users.

Moreover, when the interfaces between the ventilator 200 and the anesthesia main machine 100 are docked with each other by means of detachable insertion fit, this vertical superposition layout can be better assembled, making the overall structure more compact and smaller in size.

For example, referring to FIG. 1, in an embodiment, the first driving gas interface 111 is arranged on a top end of the main machine housing 101, and the third driving gas interface is arranged on a bottom end of the ventilator housing 210; and/or the first exhaust emission interface 113 is arranged on a top end of the main machine housing 101, and the third exhaust emission interface is arranged on a bottom end of the ventilator housing 210. When the ventilator 200 and the anesthesia main machine 100 are vertically docked with each other, gas path connection can be quickly and conveniently implemented.

Further, referring to FIGS. 3 and 6, in an embodiment, a top plate 300 is further included, wherein the top plate 300 is placed above the ventilator housing 210. The top plate 300 may be mounted on the ventilator 200, and may be used as a receiving tray for receiving and placing some articles thereon. Referring to FIGS. 3 and 7, the top plate 300 may further have a blocking structure capable of blocking interfaces of the anesthesia main machine 100. These interfaces are originally configured for docking with the ventilator 200. The anesthetic gas path system shown in FIG. 7 includes no ventilator 200, and thus the top plate 300 is used to seal the first interface group 110. Certainly, in the embodiment shown in FIG. 7, the top plate 300 may also be used as a receiving tray.

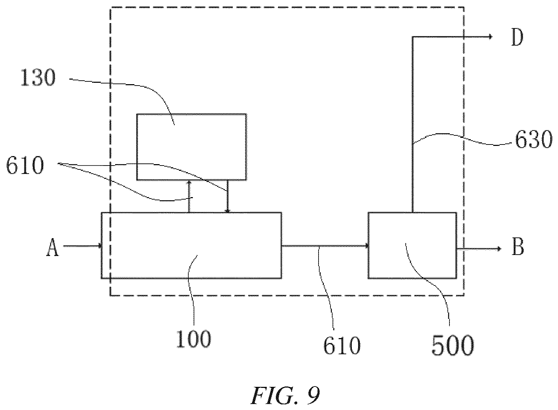

In addition, the top plate 300 is only one kind of blocking members, and in other embodiments, blocking members may be several separate plugs, etc. In the system that includes no ventilator 200 as shown in FIGS. 7 and 9, the blocking members can block the first driving gas interface 111, the second driving gas interface 121, the first exhaust emission interface 113 and/or the second exhaust emission interface 125 of the main machine housing 101 to prevent air leakage.

Further, referring to FIGS. 1, 4 and 6, in an embodiment, the first breathing circuit mechanism 400 and the anesthesia main machine 100 are arranged side by side and are detachably connected to each other. The fresh gas interface 123 and/or the second driving gas interface 121 are/is arranged on a side of the main machine housing 101, and the second fresh gas interface 421 is arranged on a side of the first breathing circuit mounting base 410 opposite to the main machine housing 101.

Certainly, when the main machine housing 101 has the second exhaust emission interface 125, the second exhaust emission interface 125 may be arranged on a side of the main machine housing 101, and the fourth exhaust emission interface 422 is arranged on a side of the first breathing circuit mounting base 410 opposite to the main machine housing 101.

In addition, the anesthesia main machine 100, the ventilator 200 and the first breathing circuit mechanism 400 may alternatively be combined and arranged in other manners, and are not limited to the foregoing structures.

On the other hand, an embodiment of the disclosure provides an anesthetic gas path system for outputting an anesthetic gas to a subject.

Referring to FIGS. 1, 5, 7 and 9, in an embodiment, the anesthetic gas path system includes components such as an anesthesia main machine 100 and a second breathing circuit mechanism 500.

The anesthesia main machine 100 is configured to supply an anesthetic gas. The anesthesia main machine 100 has a main machine housing 101 and a volatilization tank 130 for volatilizing an anesthetic drug. A first anesthetic gas passage is provided inside the main machine housing 101, and the first anesthetic gas passage communicates with the volatilization tank 130. The first anesthetic gas passage has a gas source interface (not shown) for an external gas source A to enter and a fresh gas interface 123 for outputting an anesthetic gas mixture with the anesthetic gas and air mixed, and the gas source interface and the fresh gas interface 123 are arranged on the main machine housing 101.

The second breathing circuit mechanism 500 has a second breathing circuit mounting base 510, and a second anesthetic gas passage is provided inside the second breathing circuit mounting base 510. The second anesthetic gas passage has a second fresh gas interface 521 for an anesthetic gas mixture to enter and a user interface B for leading the anesthetic gas mixture to a user. The second fresh gas interface 521 and the user interface B are arranged on the second breathing circuit mounting base 510, and the fresh gas interface 123 is in detachable insertion fit and communication with the second fresh gas interface 521, so as to deliver the anesthetic gas mixture into the user interface B.

In the anesthetic gas path system, the fresh gas interface 123 is in detachable insertion fit and communication with the second fresh gas interface 521 without excessively long connecting pipelines, which avoids externally arranged pipelines and prevents pipelines from being entangled to influence user's actions. In addition, this structure with built-in channels also makes it easier to assemble the entire system.

In an embodiment, the second breathing circuit mechanism 500 and the anesthesia main machine 100 are arranged side by side, and are detachably connected to each other, the fresh gas interface 123 is arranged on a side of the main machine housing 101, and the second fresh gas interface 521 is arranged on a side of the second breathing circuit mounting base 510 opposite to the main machine housing 101.

The disclosure has been described above with respect to specific examples, which are merely for the purpose of facilitating understanding of the disclosure and are not intended to limit the disclosure. For those skilled in the art, changes may be made to the specific embodiments described above in accordance with the concept of the disclosure.

## Claims

1. An anesthesia respiration gas path system, comprising:
an anesthesia main machine (100) configured to supply an anesthetic gas, wherein the anesthesia main machine (100) is provided with a main machine housing (101) and a volatilization tank (130) for volatilizing an anesthetic drug, a first anesthetic gas passage is provided inside the main machine housing, the first anesthetic gas passage communicates with the volatilization tank (130), the first anesthetic gas passage is provided with a gas source interface for an external gas source to enter and a fresh gas interface (123) for outputting an anesthetic gas mixture containing the anesthetic gas, and the gas source interface and the fresh gas interface(123) are arranged on the main machine housing (101);
a ventilator (200) having a ventilator housing (210), wherein a second driving gas channel and a second exhaust passage for connecting to an exhaust treatment device (D) are provided inside the ventilator housing (210), the second driving gas channel has a third driving gas interface for outputting a driving gas, the second exhaust passage has a third exhaust emission interface for emitting exhaust, and the third driving gas interface and the third exhaust emission interface are arranged on the ventilator housing (210); and
a first breathing circuit mechanism (400) having a first breathing circuit mounting base (410) and a bellows (430), wherein the bellows (430) is connected to the first breathing circuit mounting base (410), a second anesthetic gas passage and a third exhaust passage are provided inside the first breathing circuit mounting base (410), the second anesthetic gas passage has a second fresh gas interface (421) for the anesthetic gas mixture to enter and a user interface (B) for leading the anesthetic gas mixture to a subject, the third exhaust passage has a fourth exhaust emission interface (422) for emitting exhaust, the second fresh gas interface (421) , the user interface (B) and the fourth exhaust emission interface (422) are all arranged on the first breathing circuit mounting base (410), and the fresh gas interface (123) fits and communicates with the second fresh gas interface (421), so as to deliver the anesthetic gas mixture into the user interface (B);
wherein a first driving gas passage and/or a first exhaust passage are/is provided inside the main machine housing (101), the first driving gas passage has a first driving gas interface (111) and a second driving gas interface (121) which are arranged on the main machine housing (101), the first driving gas interface (111) is configured for a driving gas to enter, the second driving gas interface (121) is configured to output the driving gas, the first exhaust passage has a first exhaust emission interface (113) and a second exhaust emission interface (125) which are arranged on the main machine housing (101), the second exhaust emission interface (125) is configured for the exhaust in the first breathing circuit mechanism (400) to enter;
the third driving gas interface communicates with the first driving gas interface (111) to input the driving gas into the first driving gas passage, and the second driving gas interface (121) is configured to communicate with the bellows (430) to drive the bellows to operate; and
the fourth exhaust emission interface (422) communicates with the second exhaust emission interface (125), and the first exhaust emission interface (113) communicates with the third exhaust emission interface, to discharge the exhaust into the exhaust treatment device (D).

2. The anesthesia respiration gas path system of claim 1, wherein the ventilator (200) and the anesthesia main machine (100) are superposed one above another, and are detachably connected to each other, the first driving gas interface (111) is arranged on a top end of the main machine housing (101), and the third driving gas interface is arranged on a bottom end of the ventilator housing (210).

3. The veterinary anesthesia respiration apparatus of claim 2, wherein the first breathing circuit mechanism (400) and the anesthesia main machine (100) are arranged side by side, and are detachably connected to each other, the fresh gas interface (123) and/or the second driving gas interface (121) are/is arranged on a side of the main machine housing (101), and the second fresh gas interface (421) is arranged on a side of the first breathing circuit mounting base (410) opposite to the main machine housing (101).

4. The anesthesia respiration gas path system of claim 1, wherein the ventilator (200) and the anesthesia main machine (100) are superposed one above another, and are detachably connected to each other, the first exhaust emission interface (113) is arranged on a top end of the main machine housing (101), and the third exhaust emission interface is arranged on a bottom end of the ventilator housing (210).

5. The anesthesia respiration gas path system of claim 2, wherein the first breathing circuit mechanism (400) and the anesthesia main machine (100) are arranged side by side, and are detachably connected to each other, the fresh gas interface (123) and/or the second exhaust emission interface are/is arranged on a side of the main machine housing (101), and the fourth exhaust emission interface (422) is arranged on a side of the first breathing circuit mounting base (410) opposite to the main machine housing (101).

## Patentansprüche

1. Narkoseatemgaswegsystem, umfassend:
ein Narkosehauptgerät (100), das zum Zuführen eines Narkosegases ausgestaltet ist, wobei das Narkosehauptgerät (100) mit einem Hauptgerätgehäuse (101) und einem Verflüchtigungstank (130) zur Verflüchtigung eines Narkosemedikaments versehen ist, wobei ein erster Narkosegasdurchgang in dem Hauptgerätgehäuse vorgesehen ist, wobei der erste Narkosegasdurchgang mit dem Verflüchtigungstank (130) kommuniziert, wobei der erste Narkosegasdurchgang mit einer Gasquellenschnittstelle zum Eintritt einer externen Gasquelle und einer Frischgasschnittstelle (123) zur Ausgabe eines das Narkosegas enthaltenden Narkosegasgemischs versehen ist, und wobei die Gasquellenschnittstelle und die Frischgasschnittstelle (123) an dem Hauptgerätgehäuse (101) angeordnet sind,
ein Beatmungsgerät (200) mit einem Beatmungsgerätgehäuse (210), wobei ein zweiter Antriebsgaskanal und ein zweiter Abluftdurchgang zur Verbindung mit einer Abluftbehandlungsvorrichtung (D) in dem Beatmungsgerätgehäuse (210) vorgesehen sind, wobei der zweite Antriebsgaskanal eine dritte Antriebsgasschnittstelle zur Abgabe eines Antriebsgases hat, wobei der zweite Abluftdurchgang eine dritte Abluftemissionsschnittstelle zum Emittieren von Abluft hat und wobei die dritte Antriebsgasschnittstelle und die dritte Abluftemissionsschnittstelle an dem Beatmungsgerätgehäuse (210) angeordnet sind, und
einen ersten Atmungskreislaufmechanimus (400) mit einer ersten Atmungskreislaufmontagebasis (410) und einem Balg (430), wobei der Balg (430) mit der ersten Atmungskreislaufmontagebasis (410) verbunden ist, wobei ein zweiter Narkosegasdurchgang und ein dritter Abluftdurchgang in der ersten Atmungskreislaufmontagebasis (410) vorgesehen sind, wobei der zweite Narkosegasdurchgang eine zweite Frischgasschnittstelle (421) zum Eintritt des Narkosegasgemischs und eine Benutzerschnittstelle (B) zum Führen des Narkosegasgemischs zu einem Patienten hat, wobei der dritte Abluftdurchgang eine vierte Abluftemissionsschnittstelle (422) zum Emittieren von Abluft hat, wobei die zweite Frischgasschnittstelle (421), die Benutzerschnittstelle (B) und die vierte Abluftemissionsschnittstelle (422) an der ersten Atmungskreislaufmontagebasis (410) angeordnet sind und wobei die Frischgasschnittstelle (123) mit der zweiten Frischgasschnittstelle (421) zusammenpasst und mit dieser kommuniziert, um das Narkosegasgemisch in die Benutzerschnittstelle (B) zu liefern,
wobei ein erster Antriebsgasdurchgang und/oder ein erster Abluftdurchgang in dem Hauptgerätgehäuse (101) vorgesehen ist/sind, wobei der erste Antriebsgasdurchgang eine erste Antriebsgasschnittstelle (111) und eine zweite Antriebsgasschnittstelle (121) hat, die an dem Hauptgerätgehäuse (101) angeordnet sind, wobei die erste Antriebsgasschnittstelle (111) für den Eintritt eines Antriebsgases ausgestaltet ist, wobei die zweite Antriebsgasschnittstelle (121) zur Ausgabe des Antriebsgases ausgestaltet ist, wobei der erste Abluftdurchgang eine erste Abluftemissionsschnittstelle (113) und eine zweite Abluftemissionsschnittstelle (125) hat, die an dem Hauptgerätgehäuse (101) angeordnet sind, wobei die zweite Abluftemissionsschnittstelle (125) zum Eintritt der Abluft in den ersten Atmungskreislaufmechanimus (400) ausgestaltet ist,
wobei die dritte Antriebsgasschnittstelle mit der ersten Antriebsgasschnittstelle (111) kommuniziert, um das Antriebsgas in den ersten Antriebsgasdurchgang einzugeben, und wobei die zweite Antriebsgasschnittstelle (121) dazu ausgestaltet ist, mit dem Balg (430) zu kommunizieren, um den Betrieb des Balgs anzutreiben, und
wobei die vierte Abluftemissionsschnittstelle (422) mit der zweiten Abluftemissionsschnittstelle (125) kommuniziert und die erste Abluftemissionsschnittstelle (113) mit der dritten Abluftemissionsschnittstelle kommuniziert, um die Abluft in die Abluftbehandlungsvorrichtung (D) auszutragen.

2. Narkoseatemgaswegsystem nach Anspruch 1, wobei das Beatmungsgerät (200) und das Narkosehauptgerät (100) übereinander angeordnet und lösbar miteinander verbunden sind, wobei die erste Antriebsgasschnittstelle (111) an einem oberen Ende des Hauptgerätgehäuses (101) angeordnet ist und wobei die dritte Antriebsgasschnittstelle an einem unteren Ende des Beatmungsgerätgehäuses (210) angeordnet ist.

3. Tiermedizinisches Narkosebeatmungsgerät nach Anspruch 2, wobei der erste Atmungskreislaufmechanimus (400) und das Narkosehauptgerät (100) nebeneinander angeordnet und lösbar miteinander verbunden sind, wobei die Frischgasschnittstelle (123) und/oder die zweite Antriebsgasschnittstelle (121) an einer Seite des Hauptgerätgehäuses (101) angeordnet ist/sind und wobei die zweite Frischgasschnittstelle (421) an einer Seite der ersten Atmungskreislaufmontagebasis (410) gegenüber dem Hauptgerätgehäuse (101) angeordnet ist.

4. Narkoseatemgaswegsystem nach Anspruch 1, wobei das Beatmungsgerät (200) und das Narkosehauptgerät (100) übereinander angeordnet und lösbar miteinander verbunden sind, wobei die erste Abluftemissionsschnittstelle (113) an einem oberen Ende des Hauptgerätgehäuses (101) angeordnet ist und die dritte Abluftemissionsschnittstelle an einem unteren Ende des Beatmungsgerätgehäuses (210) angeordnet ist.

5. Narkoseatemgaswegsystem nach Anspruch 2, wobei der erste Atmungskreislaufmechanimus (400) und das Narkosehauptgerät (100) nebeneinander angeordnet und lösbar miteinander verbunden sind, wobei die Frischgasschnittstelle (123) und/oder die zweite Abluftemissionsschnittstelle an einer Seite des Hauptgerätgehäuses (101) angeordnet ist/sind und wobei die vierte Abluftemissionsschnittstelle (422) an einer Seite der ersten Atmungskreislaufmontagebasis (410) gegenüber dem Hauptgerätgehäuse (101) angeordnet ist.

## Revendications

1. Système d'acheminement de gaz respiratoire pour anesthésie, comprenant :
une machine principale d'anesthésie (100) configurée pour fournir un gaz anesthésique, dans lequel la machine principale d'anesthésie (100) est équipée d'un boîtier de machine principale (101) et d'un réservoir de volatilisation (130) pour volatiliser un médicament anesthésique, un premier passage de gaz anesthésique est prévu à l'intérieur du boîtier de machine principale, le premier passage de gaz anesthésique communique avec le réservoir de volatilisation (130), le premier passage de gaz anesthésique est doté d'une interface de source de gaz pour l'entrée d'une source de gaz externe et d'une interface de gaz frais (123) pour la sortie d'un mélange gazeux anesthésique contenant le gaz anesthésique, et l'interface de source de gaz et l'interface de gaz frais (123) sont disposées sur le boîtier de machine principale (101) ;
un ventilateur (200) présentant un boîtier de ventilateur (210), dans lequel un second canal de gaz d'entraînement et un deuxième passage d'évacuation pour un raccordement à un dispositif de traitement d'évacuation (D) sont prévus à l'intérieur du boîtier de ventilateur (210), le second canal de gaz d'entraînement présente une troisième interface de gaz d'entraînement pour sortir un gaz d'entraînement, le deuxième passage d'évacuation présente une troisième interface d'émission d'évacuation pour sortir une évacuation, et la troisième interface de gaz d'entraînement et la troisième interface d'émission d'évacuation sont disposées sur le boîtier de ventilateur (210) ; et
un premier mécanisme de circuit respiratoire (400) présentant une première base de montage de circuit respiratoire (410) et un soufflet (430), dans lequel le soufflet (430) est relié à la première base de montage de circuit respiratoire (410), un deuxième passage de gaz anesthésique et un troisième passage d'évacuation sont prévus à l'intérieur de la première base de montage de circuit respiratoire (410), le deuxième passage de gaz anesthésique présente une deuxième interface de gaz frais (421) pour l'entrée du mélange gazeux anesthésique et une interface utilisateur (B) pour diriger le mélange gazeux anesthésique vers un sujet, le troisième passage d'évacuation présente une quatrième interface d'émission d'évacuation (422) pour l'émission d'une évacuation, la deuxième interface de gaz frais (421), l'interface utilisateur (B) et la quatrième interface d'émission d'évacuation (422) sont toutes disposées sur la première base de montage de circuit respiratoire (410), et l'interface de gaz frais (123) s'adapte sur la deuxième interface de gaz frais (421) et communique avec celle-ci, de façon à délivrer le mélange gazeux anesthésique dans l'interface utilisateur (B) ;
dans lequel un premier passage de gaz d'entraînement et/ou un premier passage d'évacuation est/sont prévu(s) à l'intérieur du boîtier de machine principale (101), le premier passage de gaz d'entraînement présente une première interface de gaz d'entraînement (111) et une deuxième interface de gaz d'entraînement (121) qui sont disposées sur le boîtier de machine principale (101), la première interface de gaz d'entraînement (111) est configurée pour l'entrée d'un gaz d'entraînement, la deuxième interface de gaz d'entraînement (121) est configurée pour la sortie du gaz d'entraînement, le premier passage d'évacuation présente une première interface d'émission d'évacuation (113) et une deuxième interface d'émission d'évacuation (125) qui sont disposées sur le boîtier de machine principale (101), la deuxième interface d'émission d'évacuation (125) est configurée pour l'entrée de l'évacuation dans le premier mécanisme de circuit respiratoire (400) ;
la troisième interface de gaz d'entraînement communique avec la première interface de gaz d'entraînement (111) pour entrer le gaz d'entraînement dans le premier passage de gaz d'entraînement, et la deuxième interface de gaz d'entraînement (121) est configurée pour communiquer avec le soufflet (430) afin d'entraîner le fonctionnement du soufflet ; et
la quatrième interface d'émission d'évacuation (422) communique avec la deuxième interface d'émission d'évacuation (125), et la première interface d'émission d'évacuation (113) communique avec la troisième interface d'émission d'évacuation, pour évacuer l'évacuation dans le dispositif de traitement d'évacuation (D).

2. Système d'acheminement de gaz respiratoire pour anesthésie selon la revendication 1, dans lequel le ventilateur (200) et la machine principale d'anesthésie (100) sont superposés, et sont raccordés de manière détachable l'un à l'autre, la première interface de gaz d'entraînement (111) est disposée sur une extrémité supérieure du boîtier de machine principale (101), et la troisième interface de gaz d'entraînement est disposée sur une extrémité inférieure du boîtier de ventilateur (210).

3. Appareil respiratoire pour anesthésie vétérinaire selon la revendication 2, dans lequel le premier mécanisme de circuit respiratoire (400) et la machine principale d'anesthésie (100) sont disposés côte à côte, et sont raccordés de manière détachable l'un à l'autre, l'interface de gaz frais (123) et/ou la deuxième interface de gaz d'entraînement (121) est/sont disposée(s) sur un côté du boîtier de machine principale (101), et la deuxième interface de gaz frais (421) est disposée sur un côté de la première base de montage de circuit respiratoire (410) en regard du boîtier de machine principale (101).

4. Système d'acheminement de gaz respiratoire pour anesthésie selon la revendication 1, dans lequel le ventilateur (200) et la machine principale d'anesthésie (100) sont superposés, et sont raccordés de manière détachable l'un à l'autre, la première interface d'émission d'évacuation (113) est disposée sur une extrémité supérieure du boîtier de machine principale (101), et la troisième interface d'émission d'évacuation est disposée sur une extrémité inférieure du boîtier de ventilateur (210).

5. Système d'acheminement de gaz respiratoire pour anesthésie selon la revendication 2, dans lequel le premier mécanisme de circuit respiratoire (400) et la machine principale d'anesthésie (100) sont disposés côte à côte, et sont raccordés de manière détachable l'un à l'autre, l'interface de gaz frais (123) et/ou la deuxième interface d'émission d'évacuation est/sont disposée(s) sur un côté du boîtier de machine principale (101), et la quatrième interface d'émission d'évacuation (422) est disposée sur un côté de la première base de montage de circuit respiratoire (410) en regard du boîtier de machine principale (101).
